# EUROPEAN PATENT SPECIFICATION

(11) **EP 3 901 614 B1**
(45) Date of publication and mention of the grant of the patent: **08.02.2023**
(21) Application number: 18944056.3
(22) Date of filing: 20.12.2018
(51) Int. Cl.: G01N 21/17, A61B 5/1172, G01B 9/02

(54) **OPTICAL COHERENCE TOMOGRAPHY DEVICE**
VORRICHTUNG FÜR OPTISCHE KOHÄRENZTOMOGRAFIE
DISPOSITIF DE TOMOGRAPHIE PAR COHÉRENCE OPTIQUE

(43) Date of publication of application: 27.10.2021
(73) Proprietor: NEC Corporation, 108-8001 Tokyo (JP)
(72) Inventor: NAKAMURA Shigeru, Tokyo 108-8001 (JP)
(74) Representative: Betten & Resch
(86) International application number: PCT/JP2018/046900
(87) International publication number: WO 2020/129200

(56) References cited:
- EP-A1- 3 205 976
- WO-A1-2010/134641
- JP-A- 2011 242 177
- JP-A- 2012 042 348
- JP-A- 2012 110 575
- JP-A- 2013 007 601
- JP-A- 2017 173 305
- US-A1- 2011 150 293
- US-A1- 2012 044 499
- US-A1- 2014 002 793
- US-A1- 2016 045 106

## Description

### [Technical Field]

The disclosure relates to an optical coherence tomography device.

### [Background Art]

An optical coherence tomography (OCT) technology is a technique for performing tomography near a surface of a measurement target. The OCT technology uses interference between a scattered beam (hereinafter, also referred to as "backscattered beam") from inside of a measurement target when the measurement target is irradiated with a light beam, and a reference beam, and thereby performs tomography near a surface of the measurement target. In recent years, the OCT technology is increasingly applied to a medical diagnosis and industrial product testing.

The OCT technology uses interference between an object light beam being scattered by irradiating the measurement target, and the reference beam, and thereby identifies an optical axis-direction position, i.e., a depth-direction position of a part (optical beam scattering point) where the object light beam is scattered in the measurement target. Thus, structural data spatially decomposed in the depth direction of the measurement target are acquired. In many cases, the object light beam is not reflected by 100% only on the surface of the measurement target, but is propagated into the measurement target to some extent and then backscattered. Therefore, it is possible to acquire the structural data spatially decomposed in the depth direction of inside of the measurement target part. The OCT technology includes a time-domain OCT method (TD-OCT method) and a Fourier-domain OCT method (FD-OCT method), and the FD-OCT method is more promising in terms of high speed and high sensitivity. In the FD-OCT method, when an object light beam and a reference beam interfere with each other, an interference light spectrum in a wide wavelength band is measured and Fourier-transformed, thereby depth-direction structural data are acquired. A method for acquiring the interference light spectrum includes a spectral-domain OCT method (SD-OCT method) using a spectroscope and a swept-source OCT method (SS-OCT method) using a light source for sweeping a wavelength.

Further, the object light beam is scanned in an in-plane direction perpendicular to the depth direction of the measurement target, and thereby tomographic structural data spatially decomposed both in the in-plane direction and in the depth direction are acquired. Thus, it is possible to acquire three-dimensional tomographic structural data about the measurement target. In order to irradiate a different position of the measurement target in the in-plane direction with the object light beam, the irradiated position with one object light beam is usually scanned by a galvano scanner or the like.

The OCT technology comes into practical use as a tomography device for an ocular fundus in an ophthalmic diagnosis, and application as a non-invasive tomography device for various parts of a living body is being studied. For example, Patent Literature 1 (PTL1) discloses a technique for reading a dermal fingerprint using the OCT.

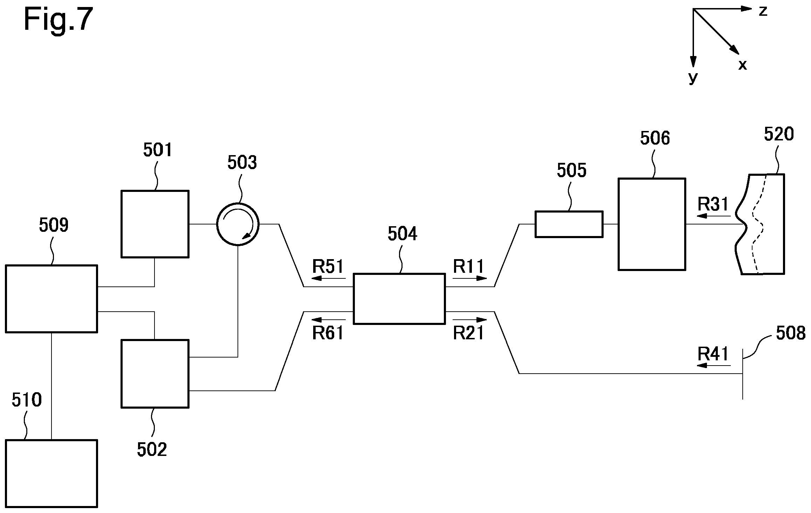

Fig. 7 illustrates a typical configuration of an optical coherence tomography device using the SS-OCT method. A wavelength-swept laser light source 501 generates a wavelength-swept optical pulse. Light emitted from the wavelength-swept laser light source 501 is split into an object light beam R11 and a reference beam R21 at a splitting/combining device 504 via a circulator 503. A measurement target 520 is irradiated with the object light beam R11 via a fiber collimator 505 and an irradiation optical system 506 including a scanning mirror and a lens. Then, an object light beam R31 to be scattered in the measurement target 520 returns to the splitting/combining device 504. Meanwhile, the reference beam R21 returns to the splitting/combining device 504 via a reference beam mirror 508. Thus, in the splitting/combining device 504, the object light beam R31 being scattered from the measurement target 520 and a reference beam R41 being reflected from the reference beam mirror 508 interfere with each other, and thereby interference light R51 and R61 are acquired. Therefore, an intensity ratio between the interference light R51 and the interference light R61 is determined by a phase difference between the object light beam R31 and the reference beam R41. The interference light R51 is input, via the circulator 503, to a balanced photodetector 502 with two optical input ports, and the interference light R61 is input directly to the balanced photodetector 502.

The intensity ratio between the interference light R51 and the interference light R61 varies in response to a change in a wavelength of light to be emitted from the wavelength-swept laser light source 501. Thus, a photoelectric conversion output at the balanced photodetector 502 can be measured as an interference light spectrum. Data indicating intensity of the backscattered beam (object light beam) at different positions in the depth direction (Z direction) can be acquired by measuring the interference light spectrum and applying Fourier transform to the measured interference light spectrum (hereinafter, an operation of acquiring data indicating the intensity of the backscattered beam (object light beam) at a certain position of the measurement target 520 in the depth direction (Z direction) is referred to as "A-scan").

Further, an irradiation position of the object light beam R11 is moved by the irradiation optical system 506, and the measurement target 520 is scanned. A two-dimensional map of intensity of the backscattered beam (object light beam) in a scanning line direction and in the depth direction can be acquired as tomographic structural data by repeating the A-scan operation while the irradiation position of the object light beam R11 is moved in the scanning line direction (X direction) by the irradiation optical system 506 and connecting measurement results (hereinafter, an operation of repeating the A-scan operation in the scanning line direction (X direction) and connecting the measurement results is referred to as "B-scan").

Furthermore, three-dimensional tomographic structural data can be acquired by repeating the B-scan operation while an irradiation position of an object light beam R1 is moved not only in the scanning line direction but also in a direction perpendicular to the scanning line (Y direction) by the irradiation optical system 506 and connecting measurement results (hereinafter, an operation of repeating the B-scan operation in the direction perpendicular to the scanning line (Y direction) and connecting the measurement results is referred to as "C-scan").

When the measurement target is a living body, it is usually difficult to perform measurement by completely fixing the living body, and it is necessary to perform the measurement at high speed. When the measurement is performed in a wide range, it is difficult to increase the speed only by scanning with a beam, and a configuration in which irradiation is performed with a plurality of object light beams is known (Patent Literature 2 (PTL2)). In addition, blood flow inside a living body can be detected by performing data processing that distinguishes a flow part from a stationary part for structural data measured multiple times in a same place, and capillary angiography and the like using that technique are known. In this case, it is also necessary to perform measurement at high speed in order to distinguish the blood flow from movement of the living body itself. A configuration in which irradiation is performed with a plurality of object light beams is known in order to perform measurement multiple times in a short time (Patent Literature 3 (PTL3)).

Patent Literature 4 (PTL4) relates to an optical imaging device that scans an ocular fundus of an eye to be examined with an optical beam and forms an image, based on a reflected light beam, and proposes that a same area of the ocular fundus of the eye to be examined is scanned in a different time with each beam of a plurality of beams with which different positions of the ocular fundus of the eye to be examined are irradiated. Further OCT devices using multiple measurement beams scanned over the object are known from documents WO 2010/134641 A1, EP 3 205 976 A1, US 2012/044499 A1 and US 2016/045106 A1.

### [Citation List]

### [Patent Literature]

[PTL1] U.S. Patent Application Publication No. 2015/0363630
[PTL2] Japanese Patent Application Laid-Open No. 2010-167253
[PTL3] Japanese Patent Application Laid-Open No. 2018-121888
[PTL4] Japanese Patent Application Laid-Open No. 2011-156035
[PTL5] U.S. Patent Application Publication No. 2009/0059971

### [Summary of Invention]

### [Technical Problem]

Irradiation with a plurality of object light beams to be used for performing wide range measurement at high speed is usually configured in such a way as to simultaneously irradiate separated places with light beams. Meanwhile, irradiation with a plurality of object light beams to be used for detecting a flow part is usually configured in such a way as to irradiate a same place with light beams multiple times in a short time. Therefore, different configurations are used.

However, for example, when an optical coherence tomography device is used for fingerprint authentication, a function of measuring a fingerprint pattern in a wide range and a function of detecting a blood flow being effective for fake finger detection are required, and use of devices with different configurations in order to achieve both functions causes an increase in size and cost.

An object of the disclosure is to provide an optical coherence tomography device that achieves both functions of wide range measurement and flow part discrimination measurement with minimal configuration modification.

### [Solution to Problem]

In order to achieve the above-mentioned object, an optical coherence tomography device according to the appended claim 1 and a corresponding method of generating an optical coherence tomographic image as defined in claim 3 are provided.

### [Advantageous Effects of Invention]

The optical coherence tomography device according to the disclosure is able to provide both functions of wide range measurement and flow part discrimination measurement with minimal configuration modification.

### [Brief Description of Drawings]

[Fig. 1] Fig. 1 is a block diagram illustrating one example of an optical coherence tomography device according to an example embodiment of a superordinate concept of the disclosure.
[Fig. 2] Fig. 2 is a block diagram illustrating one example of an optical coherence tomography device according to an example embodiment.
[Fig. 3] Fig. 3 is a diagram illustrating one example of a configuration of an irradiation optical system in the optical coherence tomography device according to the example embodiment.
[Fig. 4] Fig. 4 is a diagram illustrating one example of an object light beam scanning pattern using the irradiation optical system in the optical coherence tomography device according to the example embodiment.
[Fig. 5] Fig. 5 is a diagram illustrating one example of the configuration of the irradiation optical system in the optical coherence tomography device according to the example embodiment that is not covered by the claims.
[Fig. 6] Fig. 6 is a block diagram illustrating one example of an optical coherence tomography device according to another example embodiment.
[Fig. 7] Fig. 7 is a diagram illustrating one example of a related optical coherence tomography device.

### [Example Embodiment]

Hereinafter, example embodiments are described with reference to the drawings.

### <Example embodiment of Superordinate Concept>

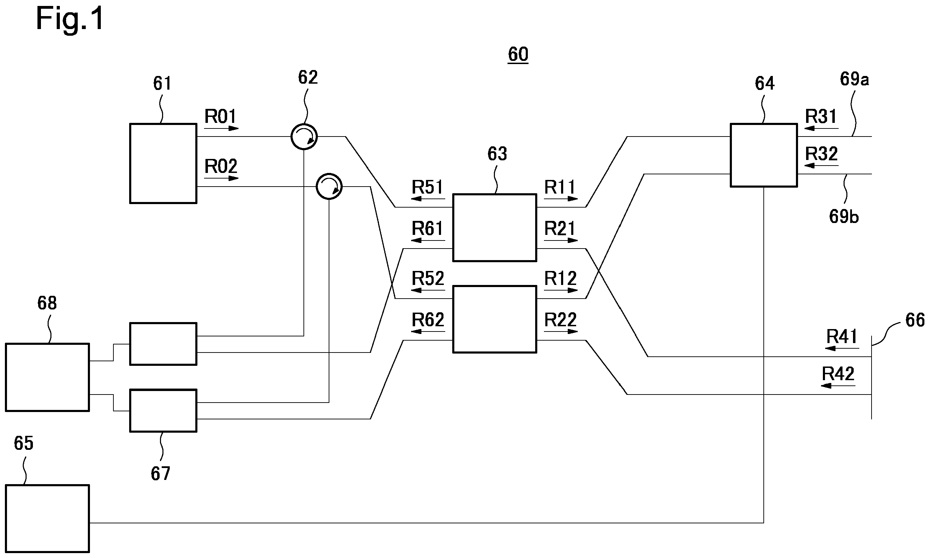

Before describing a more specific example embodiment, an optical coherence tomography device according to an example embodiment of a superordinate concept of the disclosure is described. Fig. 1 is a block diagram illustrating one example of the optical coherence tomography device according to the example embodiment of the superordinate concept of the disclosure.

An optical coherence tomography device 60 in Fig. 1 includes an optical splitting device 61, a plurality of circulators 62, a plurality of splitting/combining devices 63, an irradiation optical system 64, a reference beam mirror 66, a plurality of balanced photodetectors 67, an optical spectrum data generating means 68, a control means 65, and the like.

The optical splitting device 61 splits light incident from a laser light source or the like into a plurality of light beams R01 and R02. The plurality of light beams R01 and R02 are split into object light beams R11 and R12 and reference beams R21 and R22 by the plurality of splitting/combining devices 63 via the plurality of circulators 62.

The plurality of object light beams R11 and R12 being output from each of the splitting/combining devices 63 pass through the irradiation optical system 64, and a measurement target is irradiated with the plurality of object light beams R11 and R12 and scanned. More specifically, the irradiation optical system 64 irradiates different positions on one plane of the measurement target with each of a plurality of object light beams 69a and 69b, and scans a certain range. Herein, the irradiation optical system 64 is provided with a mechanism for controlling a distance between the object light beam 69a and the object light beam 69b.

The control means 65 controls the irradiation optical system 64 in such a way as to irradiate the different positions on one plane of the measurement target with each of the plurality of object light beams R11 and R12. Preferably, the control means 65 controls a period and a speed for scanning the measurement target by the irradiation optical system 64.

The plurality of reference beams R21 and R22 being output from each of the splitting/combining devices 63 are reflected by the reference beam mirror 66, and return to the splitting/combining devices 63.

In the splitting/combining device 63, an object light beam R31 to be scattered from the measurement target and a reference beam R41 to be reflected from the reference beam mirror 66 interfere with each other, and an interference light R51 and an interference light R61 are acquired. Similarly, in the splitting/combining device 63, an object light beam R32 to be scattered from the measurement target and a reference beam R42 to be reflected from the reference beam mirror 66 interfere with each other, and an interference light R52 and an interference light R62 are acquired.

The interference light R51 and R52 are input to the associated each of balanced photodetectors 67 via the circulators 62, and the interference light R61 and R62 are directly input to the associated each of balanced photodetectors 67. Information on a change in an intensity ratio between the interference light R51 and the interference light R61 and information on a change in an intensity ratio between the interference light R52 and the interference light R62 are each input, from each of the balanced photodetectors 67, to the optical spectrum data generating means 68.

The optical spectrum data generating means 68 generates an interference light spectrum, based on information on a wavelength change in the light incident to the optical splitting device 61 and the information on the change in the intensity ratio between the interference light R51 and R61. Similarly, the optical spectrum data generating means 68 generates the interference light spectrum, based on the information on the wavelength change in the light incident to the optical splitting device 61 and the information on the change in the intensity ratio between the interference light R52 and R62. Further, the optical spectrum data generating means 68 connects the generated interference light spectra and generates interference light spectrum data related to the measurement target.

According to the optical coherence tomography device 60 in Fig. 1, the light emitted from the laser light source or the like is split into the plurality of light beams R01 and R02 by the optical splitting device 61. Each of the light beams R01 and R02 is split into the plurality of measurement beams R11 and R12 and the plurality of reference beams R21 and R22 by the plurality of splitting/combining devices 63. The plurality of measurement beams R11 and R12 are directed to different measurement positions of the measurement target by the irradiation optical system 64. The scattered beams to be generated at each of the measurement positions are directed to each of the splitting/combining devices 63 again via the irradiation optical system 64, and are combined with the reference beams at each of the splitting/combining devices 63, and thus the interference light R51, R52, R61, and R62 are generated. The plurality of reference beams R21 and R22 to be split by each of the splitting/combining devices 63 are reflected by the reference beam mirror 66, and return to each of the splitting/combining devices 63. The interference light R51 and R52 to be generated by each of the splitting/combining devices 63 are incident to the balanced photodetectors 67 via the circulators 62, the interference light R61 and R62 are directly incident to the balanced photodetectors 67, a plurality of synthesized light beams are each spectroscopically measured, a signal processing step of performing Fourier transform processing or the like is performed, and thus the interference light spectrum data related to the measurement target is generated.

Further, according to the optical coherence tomography device 60 in Fig. 1, the distance between the plurality of object light beams 69a and 69b from the irradiation optical system 64 to the measurement target is controlled and the distance between the positions for irradiation to the measurement target with the plurality of object light beams 69a and 69b is changeable, and thus the both functions of wide range measurement and flow part discrimination measurement can be provided. Hereinafter, a more specific example embodiment is described.

### <One Example Embodiment>

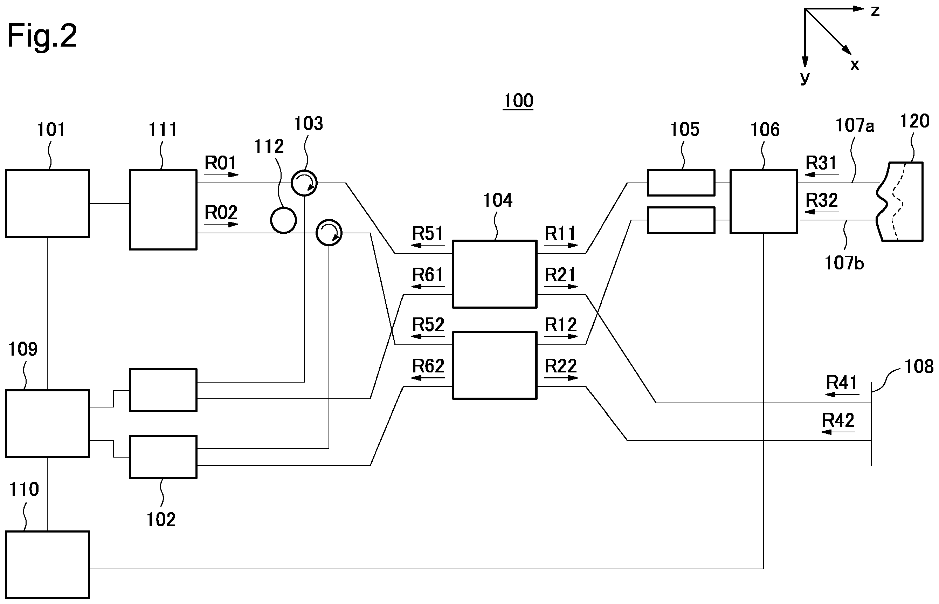

Fig. 2 is a block diagram illustrating one example of an optical coherence tomography device 100 according to one example embodiment. As illustrated in Fig. 2, the optical coherence tomography device 100 includes a wavelength-swept laser light source 101, an optical splitting device 111, a delay device 112, a plurality of circulators 103, a plurality of beam splitting/combining devices 104, a plurality of fiber collimators 105, an irradiation optical system 106, a reference beam mirror 108, a plurality of balanced photodetectors 102, an optical spectrum data generating unit 109 as one example of optical spectrum data generating means, a control unit 110 as one example of control means, and the like. Fig. 2 illustrates a case where the number of the circulators 103 is 2, the number of the beam splitting/combining devices 104 is 2, the number of the fiber collimators 105 is 2, and the number of the balanced photodetectors 102 is 2 provided in the optical coherence tomography device 100. However, the number of the circulators 103, the number of the beam splitting/combining devices 104, the number of the fiber collimators 105, and the number of the balanced photodetectors 102 provided in the optical coherence tomography device 100 according to the one example embodiment may be determined depending on the number of which light beams emitted from the wavelength-swept laser light source 101 are split in the optical splitting device 111, and are not limited to the numbers illustrated in the drawing.

The wavelength-swept laser light source 101 generates a wavelength-swept optical pulse. Specifically, the wavelength-swept laser light source 101 generates an optical pulse of which a wavelength increases from 1250 nm to 1350 nm over a duration of 10 µs. Further, the wavelength-swept laser light source 101 repeatedly generates the optical pulse at a frequency of 50kHz every 20 µs. One example of a wavelength-swept laser light source to be used for SS-OCT is described in Patent Literature 5 (PTL5).

The light emitted from the wavelength-swept laser light source 101 is split into a plurality of light beams R01 and R02 by the optical splitting device 111, and the plurality of light beams R01 and R02 pass through the plurality of circulators 103 and are split into object light beams R11 and R12 and reference beams R21 and R22 by the plurality of beam splitting/combining devices 104. As the beam splitting/combining device 104, a device using fiber fusion, a device using microoptics, or the like can be used. Note that, the delay device 112 provides a time delay to the light beam R02 with the light beam R01 as a reference. For example, in the delay device 112, a time delay of 3ns is added to the light beam R02.

The plurality of object light beams R11 and R12 to be output from the beam splitting/combining devices 104 pass through the fiber collimators 105 and the irradiation optical system 106, and a measurement target 120 is irradiated with the plurality of object light beams R11 and R12 and scanned. More specifically, the irradiation optical system 106 irradiates different positions on an X-Y plane of the measurement target 120 with a plurality of object light beams 107a and 107b and scan a certain range. The irradiation optical system 106 is provided with a mechanism for controlling a distance between the object light beam 107a and the object light beam 107b.

The object light beams 107a and 107b with which irradiate the measurement target 120 are scattered backward (in a direction opposite to the irradiation direction of the object light beam) from the measurement target 120. Then, object light beams (backscattered beams) R31 and R32 to be scattered from the measurement target 120 pass through the irradiation optical system 106 and the fiber collimators 105, and return to the beam splitting/combining devices 104.

The plurality of reference beams R21 and R22 to be output from each of the beam splitting/combining devices 104 are reflected by the reference beam mirror 108 and return to the beam splitting/combining devices 104.

Therefore, in the beam splitting/combining device 104, the object light beam R31 to be scattered from the measurement target 120 and a reference beam R41 to be reflected from the reference beam mirror 108 interfere with each other, thereby acquiring interference light R51 and interference light R61. Similarly, in the beam splitting/combining device 104, the object light beam R32 to be scattered from the measurement target 120 and a reference beam R42 to be reflected from the reference beam mirror 108 interfere with each other, thereby acquiring interference light R52 and interference light R62. Therefore, an intensity ratio between the interference light R51 and R52 and the interference light R61 and R62 is determined by a phase difference between the object light beams R31 and R32 and the reference beams R41 and R42.

The interference light R51 and R52 are input to the associated each of balanced photodetectors 102 via the circulators 103, and the interference light R61 and R62 are directly input to the associated each of balanced photodetectors 102. Then, information on a change in an intensity ratio between the interference light R51 and the interference light R61 and information on a change in an intensity ratio between the interference light R52 and the interference light R62 are each input, from each of the balanced photodetectors 102, to the optical spectrum data generating unit 109.

Note that, the balanced photodetector 102 is a photodetector in which two photo diodes are connected in series and the connection is an output (differential output). A frequency band of the balanced photodetector 102 is equal to or less than 1 GHz.

Further, optical path length of the object light beam and optical path length of the reference beam from when the object light beam R11 and the reference beam R21 are split by the beam splitting/combining device 104 until the backscattered beam R31 of the object light beam and the returned light beam R41 of the reference beam are combined again are approximately equal. When the optical path length greatly differs between the object light beam and the reference beam, a frequency difference (wavelength difference) between the object light beam R31 and the reference beam R41 that interfere with each other in the beam splitting/combining device 104 becomes larger than the frequency band of the balanced photodetector 102, and it becomes difficult to detect the intensity ratio between the interference light R51 and the interference light R61 that reflects a phase difference between the object light beam R31 and the reference beam R41. Further, optical path length of the object light beam and optical path length of the reference beam from when the object light beam R12 and the reference beam R22 are split by the beam splitting/combining device 104 until the backscattered beam R32 of the object light beam and the returned light beam R42 of the reference beam are combined again are approximately equal. When the optical path length greatly differs between the object light beam and the reference beam, a frequency difference (wavelength difference) between the object light beam R32 and the reference beam R42 that interfere with each other in the beam splitting/combining device 104 becomes larger than the frequency band of the balanced photodetector 102, and it becomes difficult to detect the intensity ratio between the interference light R51 and the interference light R61 that reflects a phase difference between the object light beam R31 and the reference beam R41.

The optical spectrum data generating unit 109 generates interference light spectrum data, based on information on a wavelength change in the light emitted from the wavelength-swept laser light source 101 and the information on the change in the intensity ratio between the interference light R51 and R61. Similarly, the optical spectrum data generating unit 109 generates the interference light spectrum data, based on the information on the wavelength change in the light emitted from the wavelength-swept laser light source 101 and the information on the change in the intensity ratio between the interference light R52 and R62. Further, the optical spectrum data generating unit 109 inputs the generated interference light spectrum data to the control unit 110.

The control unit 110 controls each unit of the optical coherence tomography device 100.

The control unit 110 controls the irradiation optical system 106 in such a way as to irradiate different positions on the X-Y plane of the measurement target 120 with each of the plurality of object light beams R11 and R12.

Further, the control unit 110 controls a period and a speed for scanning the measurement target 120 by the irradiation optical system 106.

Further, the control unit 110 applies Fourier transform to the interference light spectrum to be generated by the optical spectrum data generating unit 109, and acquires data indicating the intensity of a backscattered beam (object light beam) at a different position in a depth direction (Z direction) of the measurement target 120 (A-scan). More specifically, the interference light spectrum of a center wavelength λ0 and the number of sample points N in a wavelength range Δλ is acquired by A-scan, and the control unit 110 applies discrete Fourier transform to the interference light spectrum, thereby acquiring the depth-direction structural data of which unit of length is λ0²/Δλ.

Further, the control unit 110 generates two-dimensional tomographic structural data by connecting measurement results acquired by repeating the A-scan operation while the irradiation positions with the object light beams R11 and R12 are moved in a scanning line direction (B-scan). Herein, the scanning line direction is at least either of the X and the Y directions.

Further, the control unit 110 generates three-dimensional tomographic structural data in X, Y, and Z directions by connecting measurement results acquired by repeating the B-scan operation while the irradiation positions with the object light beams R11 and R12 are moved in the scanning line direction and the direction perpendicular to the scanning line (C-scan).

Further, the control unit 110 performs, when the optical coherence tomography device operates in such a way as to measure a wide range at high speed, processing of connecting a plurality of 3D structural data (three-dimensional structural data) acquired by scanning the plurality of object light beams.

Further, the control unit 110 performs, when the optical coherence tomography device operates in such a way as to detect a flow part, comparison analysis of 3D structural data acquired at a same place at different times by scanning the plurality of object light beams.

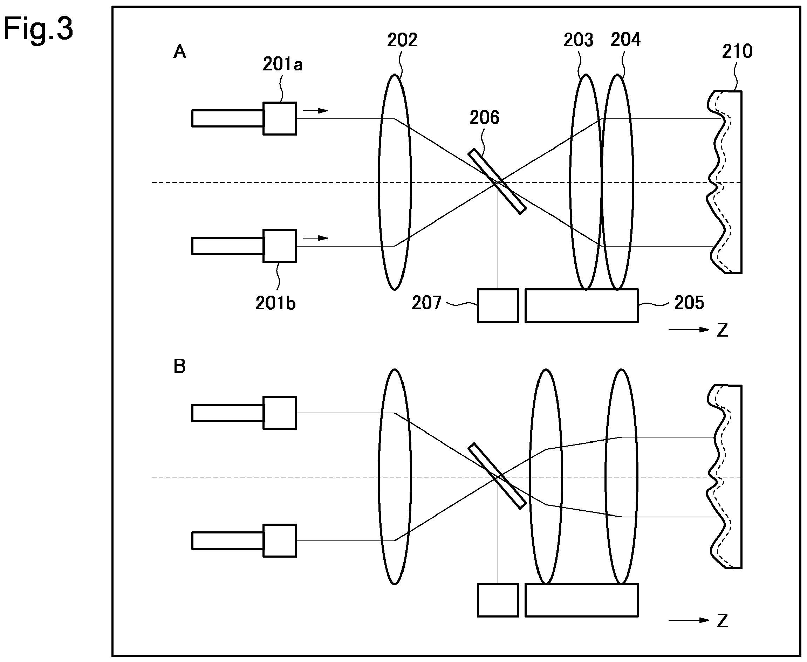

Fig. 3 is a diagram illustrating the configuration, according to the invention, of the irradiation optical system that irradiates a measurement target with a plurality of object light beams. The irradiation optical system in Fig. 3 includes a lens 202 as one example of a front lens system, a galvano scanner 206, two lenses 203 and 204 as one example of a rear lens system, a galvano scanner control unit, and a lens control unit. Object light beams propagating from a plurality of beam splitting/combining devices through fibers are emitted from a plurality of fiber collimators 201, pass through the lens 202 and become collimated light beams, and cross after propagating a certain distance. The galvano scanner 206 is set at a point where the object light beams cross, thereby enabling scanning of the object light beams. The galvano scanner 206 is controlled by a galvano scanner controller 207 as one example of the galvano scanner control unit. Further, the plurality of object light beams pass through the two lenses 203 and 204 and are each condensed on points that are separated by a certain distance near a surface of a measurement target 210. Lens positions of the two lenses 203 and 204 are controlled by a lens controller 205 as one example of the lens control unit along a direction parallel to the above Z direction of the measurement target 120.

When a distance between the two lenses 203 and 204 is short, distant positions on the measurement target are irradiated with the plurality of object light beams as a state A illustrated in Fig. 3. When the distance between the two lenses 203 and 204 is long, close positions on the measurement target are irradiated with the plurality of object light beams as a state B illustrated in Fig. 3.

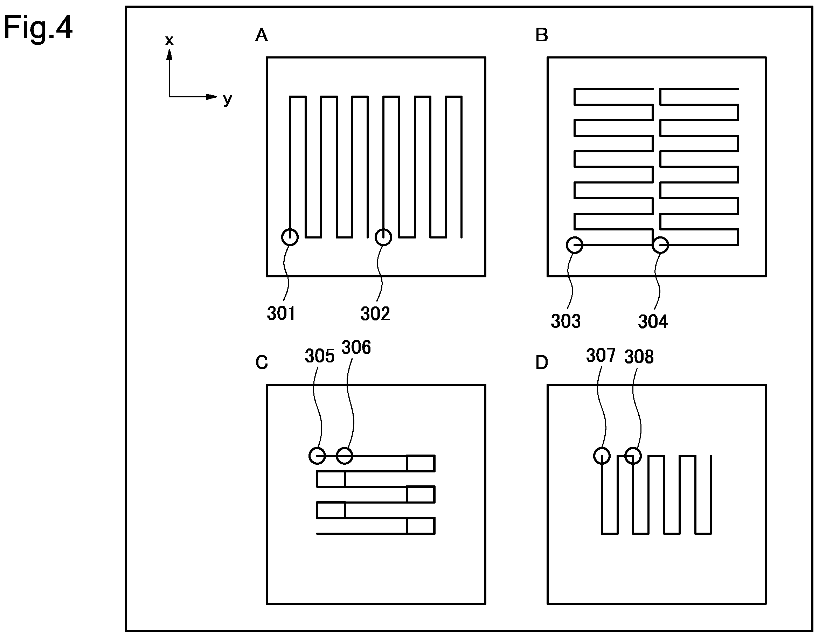

Fig. 4 is a diagram illustrating an example of a scanning pattern of the object light beam using the irradiation optical system.

Scanning A in Fig 4 or scanning B in Fig. 4 illustrates an example of a scanning pattern of which distant positions on the measurement target are irradiated with the plurality of object light beams, and the object light beams are scanned, thereby measuring a wide range at high speed. In the scanning A of Fig. 4, initial positions of irradiation points with two object light beams on the measurement target are 301 and 302, and scanning are performed at high speed in the X direction by the control of the galvano scanner as illustrated. In the scanning B of Fig. 4, initial positions of irradiation points with the two object light beams on the measurement target are 303 and 304, and the scanning are performed by the control of the galvano scanner as illustrated. In both cases, the plurality of object light beams are scanned simultaneously over different areas, thereby enabling measuring a wide range at high speed.

Scanning C in Fig 4 or scanning D in Fig. 4 illustrates an example of a scanning pattern of which close positions on the measurement target are irradiated with the plurality of object light beams, and the object light beams are scanned, thereby measuring a wide range at high speed. In the scanning C of Fig. 4, initial positions of irradiation points with two object light beams on the measurement target are 305 and 306, and scanning are performed at high speed in the X direction by the control of the galvano scanner as illustrated. In the scanning D of Fig. 4, initial positions of irradiation points with the two object light beams on the measurement target are 307 and 308, and the scanning are performed by the control of the galvano scanner as illustrated. In both cases, the plurality of object light beams are scanned at a same place multiple times in a short time, thereby enabling discrimination of a flow part.

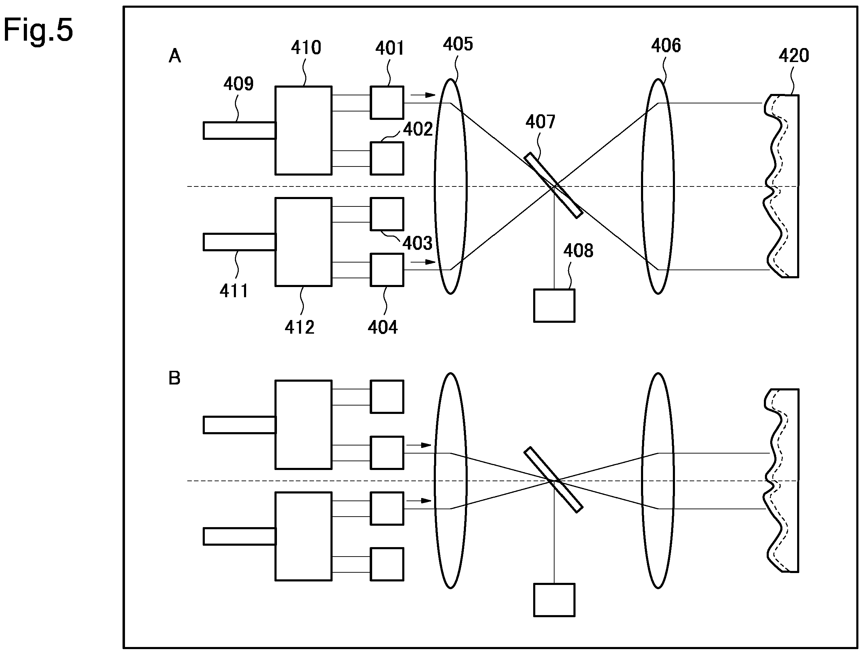

Fig. 5 is a diagram illustrating an example, not covered by the claims, of a configuration of the irradiation optical system that irradiates the measurement target with the plurality of object light beams. The irradiation optical system in Fig. 5 includes fiber collimators 401, 402, 403, and 404, a lens 405 as one example of the front lens system, a galvano scanner 407, a lens 406 as one example of the rear lens system, a galvano scanner controller 408, and optical switches 410 and 412. A plurality of fibers 409 and 411 that propagate the object light beams from the plurality of beam splitting/combining devices are each connected to the optical switches 410 and 412. The fiber collimators 401 and 402 are arranged in such a way as to direct light propagating through the fiber 409 to different positions in a radial direction of the lens 405. Similarly, the fiber collimators 404 and 403 are arranged in such a way as to direct light propagating through the fiber 411 to different positions in the radial direction of the lens 405. The optical switch 410 selects whether the object light beam propagating through the fiber 409 is passed through the fiber collimator 401 or the fiber collimator 402. The optical switch 412 selects whether the object light beam propagating through the fiber 411 is passed through the fiber collimator 403 or the fiber collimator 404. The plurality of object light beams to be emitted from the plurality of fiber collimators pass through the lens 405 and become collimated light beams, and cross after propagating a certain distance. The galvano scanner 407 is set at a point where the object light beams cross, thereby enabling scanning of the object light beams. The galvano scanner 407 is controlled by the galvano scanner controller 408 as one example of the galvano scanner control unit. Further, the plurality of object light beams pass through the lens 406 and are each condensed on points that are separated by a certain distance near a surface of a measurement target 420.

When 401 and 404 are selected as a fiber collimator for object light beam emission using the optical switches 410 and 412, distant positions on the measurement target are irradiated with the plurality of object light beams as a state A illustrated in Fig. 5. When 402 and 403 are selected as the fiber collimator for object light beam emission using the optical switches 410 and 412, close positions on the measurement target are irradiated with the plurality of object light beams as a state B illustrated in Fig. 5.

According to the optical coherence tomography device 100 according to the above-described one example embodiment, the distance between the plurality of object light beams 107a and 107b from the irradiation optical system 106 to the measurement target 120 is controlled and the distance between the positions for irradiation to the measurement target with the plurality of object light beams 107a and 107b is changeable, and thus the both functions of wide range measurement and flow part discrimination measurement can be provided.

In other words, the wide range measurement at high speed can be achieved by configuring in such a way as to control the distance between the plurality of object light beams 107a and 107b from the irradiation optical system 106 to the measurement target 120 and simultaneously irradiate distant positions on the measurement target 120 with light beams. Further, the flow part discrimination measurement can be achieved by configuring in such a way as to control the distance between the plurality of object light beams 107a and 107b from the irradiation optical system 106 to the measurement target 120 and irradiate a same place with light beams multiple times in a short time.

### <Another Example Embodiment>

As described above, the number of the circulators 103, the number of the beam splitting/combining devices 104, the number of the fiber collimators 105, and the number of the balanced photodetectors 102 provided in the optical coherence tomography device 100 according to the one example embodiment may be determined depending on the number of which light beams emitted from the wavelength-swept laser light source 101 are split in the optical splitting device 111, and are not limited to the numbers illustrated in Fig. 2.

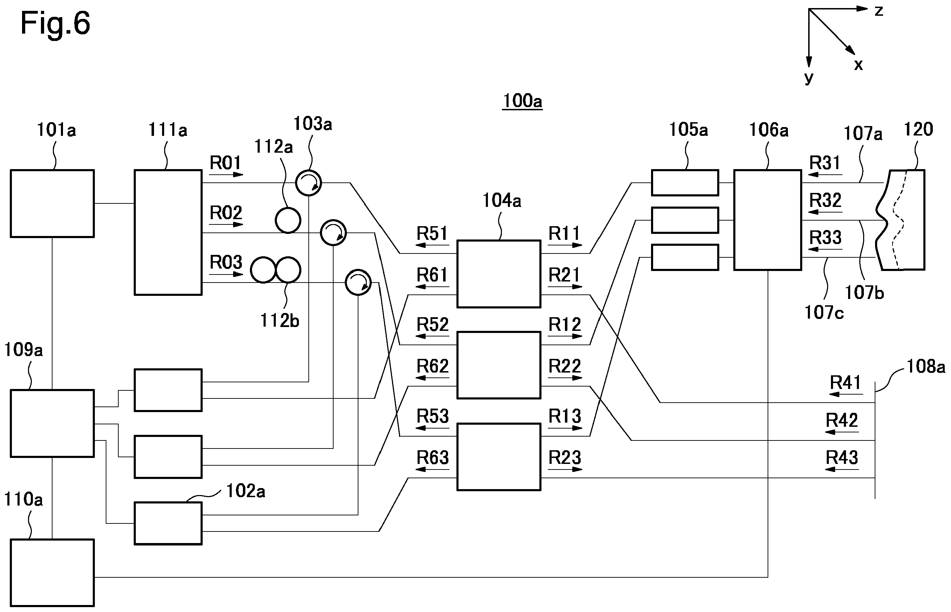

Fig. 6 is a block diagram illustrating an optical coherence tomography device 100a according to another example embodiment. The optical coherence tomography device 100a illustrated in Fig. 6 includes a wavelength-swept laser light source 101a, an optical splitting device 111a, delay devices 112a and 112b, a plurality of circulators 103a, a plurality of beam splitting/combining devices 104a, a plurality of fiber collimators 105a, an irradiation optical system 106a, a reference beam mirror 108a, a plurality of balanced photodetectors 102a, an optical spectrum data generating unit 109a as one example of optical spectrum data generating means, a control unit 110a as one example of control means, and the like. Fig. 6 illustrates a case where the number of the circulators 103a is 3, the number of the beam splitting/combining devices 104a is 3, the number of the fiber collimators 105a is 3, and the number of the balanced photodetectors 102a is 3 provided in the optical coherence tomography device 100a. Note that, the delay devices 112a and 112b provide a time delay for light beams R02 and R03 with the light beam R01 as a reference. For example, a time delay of 3ns is added in the delay device 112a and a time delay of 6ns is added in the delay device 112b.

The control unit 110a in Fig. 6 controls each unit of the optical coherence tomography device 100a, similarly to the control unit 110 in Fig.2. The control unit 110a in Fig.6 controls the irradiation optical system 106a in such a way as to irradiate different positions on a X-Y plane of a measurement target 120 with each of a plurality of object light beams R11, R12, and R13. Further, the control unit 110a in Fig. 6 controls a period and a speed for scanning the measurement target 120 by the irradiation optical system 106a.

Further, the control unit 110a in Fig.6 applies Fourier transform to an interference light spectrum to be generated by the optical spectrum data generating unit 109a, and acquires data indicating an intensity of a backscattered beam (object light beam) at a different position in a depth direction (Z direction) of the measurement target 120 (A-scan).

Further, the control unit 110a in Fig. 6 generates two-dimensional tomographic structural data by connecting measurement results acquired by repeating the A-scan operation while irradiation positions with the object light beams R11, R12 and R13 are moved in a scanning line direction (B-scan).

Further, the control unit 110 generates three-dimensional tomographic structural data in X, Y, and Z directions by connecting measurement results acquired by repeating the B-scan operation while the irradiation positions with the object light beams R11 and R12 are moved in the scanning line direction and a direction perpendicular to the scanning line (C-scan).

Further, the control unit 110a in Fig. 6 performs, when the optical coherence tomography device 100a operates in such a way as to measure a wide range at high speed, processing of connecting a plurality of 3D structural data (three-dimensional structural data) acquired by scanning the plurality of object light beams.

Further, the control unit 110 in Fig. 6 performs, when the optical coherence tomography device 100a operates in such a way as to detect a flow part, comparison analysis of 3D structural data acquired at a same place at different times by scanning the plurality of object light beams.

According to the optical coherence tomography device 100a illustrated in Fig. 6, in the beam splitting/combining device 104a, an object light beam R31 to be scattered from the measurement target 120 and a reference beam R41 to be reflected from the reference beam mirror 108a interfere with each other, thereby acquiring interference light R51 and interference light R61. Similarly, in the beam splitting/combining device 104a, an object light beam R32 to be scattered from the measurement target 120 and a reference beam R42 to be reflected from the reference beam mirror 108a interfere with each other, thereby acquiring interference light R52 and interference light R62. Similarly, in the beam splitting/combining device 104a, an object light beam R33 to be scattered from the measurement target 120 and a reference beam R43 to be reflected from the reference beam mirror 108a interfere with each other, thereby acquiring interference light R53 and interference light R63. Therefore, an intensity ratio between the interference light R51, R52, and R53 and the interference light R61, R62, and R63 is determined by a phase difference between the object light beams R31, R32, and R33 and the reference beams R41, R42, and R43.

The interference light R51, R52, and R53 are input to the associated each of balanced photodetectors 102a via the circulators 103a, and the interference light R61, R62, and R63 are directly input to the associated each of balanced photodetectors 102a. Then, information on a change in the intensity ratio between the interference light R51 and the interference light R61, information on a change in the intensity ratio between the interference light R52 and the interference light R62, and information on a change in the intensity ratio between the interference light R53 and the interference light R63 are each input, from each of the balanced photodetectors 102a, to the optical spectrum data generating unit 109a.

In the optical coherence tomography device 100a illustrated in Fig.6, similarly to the optical coherence tomography device 100 according to the one example embodiment, a distance among the positions for irradiation to the measurement target 120 with the plurality of object light beams is changeable, and thus, the both functions of wide range measurement and flow part discrimination measurement can be provided with minimal configuration modification.

In other words, the wide range measurement at high speed can be achieved by configuring in such a way as to control the distance among the plurality of object light beams 107a, 107b, and 107c from the irradiation optical system 106a to the measurement target 120 and simultaneously irradiate distant positions on the measurement target 120 with light beams. Further, the flow part discrimination measurement can be achieved by configuring in such a way as to control the distance among the plurality of object light beams 107a, 107b, and 107c from the irradiation optical system 106 to the measurement target 120 and irradiate a same place with light beams multiple times in a short time.

### <Summary of Example Embodiment>

The above-described one example embodiment can be summarized as follows. In an optical coherence tomography device, irradiation with a plurality of object light beams is useful for performing wide range measurement at high speed or performing measurement for discriminating a flow part such as blood flow, however the use of another device for performing both of the measurement results in an increase in size and cost.

In the optical coherence tomography device 100 according to the above-described one example embodiment, the wavelength-swept laser light source 101 generates a wavelength-swept optical pulse. Light emitted from the wavelength-swept laser light source 101 is split into a plurality of light beams by the optical splitting device 111, and the plurality of light beams are split into an object light beam and a reference beam by the each of beam splitting/combining devices 104. The plurality of object light beams pass through the fiber collimator 105 and the irradiation optical system 106 constituted of a scan mirror and a lens, the measurement target 120 is irradiated with the plurality of object light beams as the object light beams 107a and 107b, and backscattered beams return to each of the beam splitting/combining devices 104. On the other hand, the reference beam returns to the beam splitting/combining device 104 via the reference beam mirror 108. Thus, interference between the object light beam and the reference beam occurs, a measurement value of interference light intensity is acquired by photoelectric conversion in the balanced photodetector 102 with two optical input ports, and 3D structural data (three-dimensional structural data) is calculated in the control unit 110.

Herein, in the optical coherence tomography device 100 according to the one example embodiment, the irradiation optical system that changes a distance between the object light beams 107a and 107b is used. When wide range measurement at high speed is performed, distant positions on the measurement target are irradiated with the plurality of object light beams. In this case, wide range structural data are acquired by connecting pieces of structural data being acquired by simultaneously scanning different area. When the measurement for discriminating a flow part such as blood flow is performed, close positions on the measurement target are irradiated with the plurality of object light beams and are scanned in such a way as to irradiate a same place multiple times in a short time. In this case, a flow part is detected from the structural data acquired at the same place at different times.

Therefore, in the optical coherence tomography device 100 according to the one example embodiment, the irradiation optical system includes a mechanism for changing, on the measurement target, positions of the object light beams 107a and 107b with which irradiate different positions on the measurement target, and thus, the both functions of wide range measurement and flow part discrimination measurement can be provided with minimal configuration modification.

### [Reference signs List]

- 100: Optical coherence tomography device
- 101: Wavelength-swept laser light source
- 102: Balanced photodetector
- 103: Circulator
- 104: Beam splitting/combining device
- 105: Fiber collimator
- 106: Irradiation optical system constituted of scan mirror and lens
- 107: Object light beam
- 108: Reference beam mirror
- 109: Optical spectrum data generating unit
- 110: Control unit
- 111: Optical splitting device
- 112: Delay device
- 120: Measurement target
- 201: Fiber collimator
- 202 to 204: Lens
- 205: Lens controller
- 206: Galvano scanner
- 207: Galvano scanner controller
- 210: Measurement target
- 401 to 404: Fiber collimator
- 405 to 406: Lens
- 407: Galvano scanner
- 408: Galvano scanner controller
- 410, 412: Optical switch
- 420: Measurement target

## Claims

1. An optical coherence tomography device comprising:
split beam generating means (61; 111) for splitting light emitted from a single wavelength-swept laser light source (101) into at least four split beams and outputting the split beams;
object light beam irradiating means (64; 106) for irradiating different positions of a measurement target with object light beams (107a, 107b; 107c) being at least two of the at least four split beams through a mechanism capable of changing a position of each of the object light beams (107a, 107b; 107c) on the measurement target;
reference beam irradiating means (63; 104) for irradiating a reference beam mirror (66; 108) with at least two of the at least four split beams that are not the object light beams (107a, 107b; 107c), as reference beams (R41, R42; R43); and
optical spectrum data generating means (68; 109) for acquiring depth-direction structural data about the measurement target from interference light (R51, R52, R61, R62; R53, R63) to be acquired by causing each of the reference beams (R41, R42; R43) reflected by the reference beam mirror (66; 108) to interfere with a corresponding one of the object light beams (R31, R32; R33) reflected or scattered by the measurement target,
**characterized in that**
the object light beam irradiating means (64; 106) includes
a front lens system (202) being set in such a way that the object light beams (107a, 107b; 107c) are collimated and cross at one point,
an optical beam scanner (206) being placed at a position in which the object light beams (107a, 107b; 107c) cross, and
a rear lens system (203, 204) that causes the object light beams (107a, 107b; 107c) passing through the light beam scanner (206) to be condensed on the measurement target, and
changes, on the measurement (107a, 107b; 107c) target, a position of each of object light beams (107a, 107b; 107c) with which different positions of the measurement target are irradiated, and
wherein the optical coherence tomography device further comprises
a mechanism for causing a lens of the rear lens system (203, 204) for the object light beam irradiating means (64; 106) to move in a direction parallel to a depth direction of the measurement target.

2. The optical coherence tomography device according to claim 1, wherein
the optical spectrum data generating means (68, 109) generates information on a change in an intensity ratio between first acquired interference light (R51, R52; R53) and second acquired interference light (R61, R62; R63), and
the optical coherence tomography device further comprises control means for acquiring depth-direction structural data about the measurement target, based on the generated information.

3. A method of generating an optical coherence tomographic image by an optical coherence tomography device, comprising:
splitting light emitted from a single wavelength-swept laser light source (101) into at least four split beams and outputting the split beams;
irradiating different positions of a measurement target with object light beams (107a, 107b; 107c) being at least two of the at least four split beams by adjusting a position of each of the object light beams (107a, 107b; 107c) on the measurement target, and also irradiating a reference beam mirror (66; 108) with at least two of the at least four split beams that are not the object light beams (107a, 107b; 107c), as reference beams (R41, R42; R43); and
acquiring depth-direction structural data about the measurement target from interference light (R51, R52, R61, R62; R53, R63) to be acquired by causing each of the reference beams (R41, R42; R43) reflected by the reference beam mirror (66; 108) to interfere with a corresponding one of the object light beams (R31, R32; R33) reflected or scattered by the measurement target,
**characterized in that**
the optical coherence tomography device includes
a front lens system (202) being set in such a way that the object light beams (107a, 107b; 107c) are collimated and cross at one point,
an optical beam scanner (206) being placed at a position in which the object light beams (107a, 107b; 107c) cross, and
a rear lens system (203, 204) that causes the object light beams (107a, 107b; 107c) passing through the light beam scanner (206) to be condensed on the measurement target, and
the method further comprises changing, on the measurement target, a position of each of object light beams (107a, 107b; 107c) with which different positions of the measurement target are irradiated,
the method further comprising
adjusting a distance among irradiated positions on the measurement target with the object light beams (107a, 107b; 107c) by causing a lens of the rear lens system (203, 204) in the optical coherence tomography device to move in a direction parallel to a depth direction of the measurement target.

4. The method of generating an optical coherence tomographic image according to claim 3, wherein
information on a change in an intensity ratio between first acquired interference light (R51, R52; R53) and second acquired interference light (R61, R62; R63) is generated, and
the depth-direction structural data about the measurement target is acquired, based on the generated information.

## Patentansprüche

1. Vorrichtung für optische Kohärenztomographie, umfassend:
eine Teilstrahl-Erzeugungseinrichtung (61; 111) zum Teilen von von einer einzigen wellenlängenvariablen Laserlichtquelle (101) emittiertem Licht in wenigstens vier Teilstrahlen und Ausgeben der Teilstrahlen;
eine Objektlichtstrahl-Bestrahlungseinrichtung (64; 106) zum Bestrahlen unterschiedlicher Positionen eines Messziels mit Objektlichtstrahlen (107a, 107b; 107c), die wenigstens zwei der wenigstens vier Teilstrahlen sind, durch einen Mechanismus, der eine Position von jedem der Objektlichtstrahlen (107a, 107b; 107c) auf dem Messziel ändern kann;
eine Referenzstrahl-Bestrahlungseinrichtung (63; 104) zum Bestrahlen eines Referenzstrahlspiegels (66; 108) mit wenigstens zwei der wenigstens vier Teilstrahlen, die nicht die Objektlichtstrahlen (107a, 107b; 107c) sind, als Referenzstrahlen (R41, R42; R43); und
eine Erzeugungseinrichtung für Daten des optischen Spektrums (68; 109) zum Erlangen von Tiefenrichtungsstrukturdaten über das Messziel aus durch Veranlassen, dass jeder der durch den Referenzstrahlspiegel (66; 108) reflektierten Referenzstrahlen (R41, R42; R43) mit einem entsprechenden der durch das Messziel reflektierten oder gestreuten Objektlichtstrahlen (R31, R32; R33) interferiert, zu erlangendem Interferenzlicht (R51, R52, R61, R62; R53, R63),
**dadurch gekennzeichnet, dass**
die Objektlichtstrahl-Bestrahlungseinrichtung (64; 106) folgendes enthält:
ein Vorderlinsensystem (202), das auf derartige Weise eingestellt ist, dass die Objektlichtstrahlen (107a, 107b; 107c) kollimiert sind und sich an einem Punkt kreuzen,
einen optischen Strahlscanner (206), der bei einer Position platziert ist, bei welcher sich die Objektlichtstrahlen (107a, 107b; 107c) kreuzen, und
ein Hinterlinsensystem (203, 204), das veranlasst, dass die durch den Lichtstrahlscanner (206) laufenden Objektlichtstrahlen (107a, 107b; 107c) auf dem Messziel komprimiert werden, und
auf dem Ziel der Messung (107a, 107b; 107c) eine Position von jedem von Objektlichtstrahlen (107a, 107b; 107c) ändert, mit welchen unterschiedliche Positionen des Messziels bestrahlt werden, und
wobei die Vorrichtung für optische Kohärenztomographie weiterhin folgendes umfasst:
einen Mechanismus zum Veranlassen, dass sich eine Linse des Hinterlinsensystems (203, 204) für die Objektlichtstrahl-Bestrahlungseinrichtung (64; 106) in einer Richtung parallel zu einer Tiefenrichtung des Messziels bewegt.

2. Vorrichtung für optische Kohärenztomographie nach Anspruch 1, wobei
die Erzeugungseinrichtung für Daten des optischen Spektrums (68; 109) Information über eine Änderung bei einem Intensitätsverhältnis zwischen erstem erlangten Interferenzlicht (R51, R52; R53) und zweitem erlangten Interferenzlicht (R61, R62; R63) erzeugt, und
die Vorrichtung für optischen Kohärenztomographie weiterhin eine Steuereinrichtung zum Erlangen von Tiefenrichtungsstrukturdaten über das Messziel basierend auf der erzeugten Information umfasst.

3. Verfahren zum Erzeugen eines Bilds mittels optischer Kohärenztomographie durch eine Vorrichtung für optischen Kohärenztomographie, umfassend:
Teilen von von einer einzigen wellenlängenvariablen Laserlichtquelle (101) emittiertem Licht in wenigstens vier Teilstrahlen und Ausgeben der Teilstrahlen;
Bestrahlen unterschiedlicher Positionen eines Messziels mit Objektlichtstrahlen (107a, 107b; 107c), die wenigstens zwei der wenigstens vier Teilstrahlen sind, durch Einstellen einer Position von jedem der Objektlichtstrahlen (107a, 107b; 107c) auf dem Messziel, und auch Bestrahlen eines Referenzstrahlspiegels (66; 108) mit wenigstens zwei der wenigstens vier Teilstrahlen, die nicht die Objektlichtstrahlen (107a, 107b; 107c) sind, als Referenzstrahlen (R41, R42; R43); und
Erlangen von Tiefenrichtungsstrukturdaten über das Messziel aus durch Veranlassen, dass jeder der durch den Referenzstrahlspiegel (66; 108) reflektierten Referenzstrahlen (R41, R42; R43) mit einem entsprechenden der durch das Messziel reflektierten oder gestreuten Objektlichtstrahlen (R31, R32; R33) interferiert, zu erlangendem Interferenzlicht (R51, R52, R61, R62; R53, R63),
**dadurch gekennzeichnet, dass**
die Vorrichtung für optische Kohärenztomographie folgendes enthält:
ein Vorderlinsensystem (202), das auf derartige Weise eingestellt ist, dass die Objektlichtstrahlen (107a, 107b; 107c) kollimiert sind und sich an einem Punkt kreuzen,
einen optischen Strahlscanner (206), der bei einer Position platziert ist, bei welcher sich die Objektlichtstrahlen (107a, 107b; 107c) kreuzen, und
ein Hinterlinsensystem (203, 204), das veranlasst, dass die durch den Lichtstrahlscanner (206) laufenden Objektlichtstrahlen (107a, 107b; 107c) auf dem Messziel komprimiert werden, und
das Verfahren weiterhin ein Ändern einer Position von jedem von Objektlichtstrahlen (107a, 107b; 107c) auf dem Messziel umfasst, mit welchen unterschiedliche Positionen des Messziels bestrahlt werden,
wobei das Verfahren weiterhin folgendes umfasst:
Einstellen eines Abstands unter bestrahlten Positionen auf dem Messziel mit den Objektlichtstrahlen (107a, 107b; 107c) durch Veranlassen, dass sich eine Linse des Hinterlinsensystems (203, 204) in der Vorrichtung für optische Kohärenztomographie in einer Richtung parallel zu einer Tiefenrichtung des Messziels bewegt.

4. Verfahren zum Erzeugen eines Bilds mittels optischer Kohärenztomographie nach Anspruch 3, wobei
Information über eine Änderung bei einem Intensitätsverhältnis zwischen erstem erlangten Interferenzlicht (R51, R52; R53) und zweitem erlangten Interferenzlicht (R61, R62; R63) erzeugt wird, und
die Tiefenrichtungsstrukturdaten über das Messziel basierend auf der erzeugten Information erlangt wird.

## Revendications

1. Dispositif de tomographie par cohérence optique comprenant :
des moyens de génération de faisceaux divisés (61; 111) pour diviser une lumière émise depuis une source de lumière laser à balayage de longueur d'onde unique (101) en au moins quatre faisceaux divisés et pour délivrer les faisceaux divisés ;
des moyens d'irradiation de faisceaux lumineux objets (64; 106) pour irradier différentes positions d'une cible de mesure avec des faisceaux lumineux objets (107a, 107b ; 107c) étant au moins deux parmi les au moins quatre faisceaux divisés par l'intermédiaire d'un mécanisme capable de changer une position de chacun des faisceaux lumineux objets (107a, 107b ; 107c) sur la cible de mesure ;
des moyens d'irradiation de faisceaux de référence (63 ; 104) pour irradier un miroir de faisceaux de référence (66 ; 108) avec au moins deux parmi les au moins quatre faisceaux divisés qui ne sont pas les faisceaux lumineux objets (107a, 107b ; 107c), en tant que faisceaux de référence (R41, R42 ; R43) ; et
des moyens de génération de données de spectre optique (68 ; 109) pour acquérir des données structurelles dans la direction de profondeur en ce qui concerne la cible de mesure depuis une lumière d'interférence (R51, R52, R61, R62 ; R53, R63) à acquérir en amenant chacun des faisceaux de référence (R41, R42 ; R43) réfléchis par le miroir de faisceaux de référence (66 ; 108) à interférer avec l'un correspondant des faisceaux lumineux objets (R31, R32 ; R33) réfléchis ou diffusés par la cible de mesure,
**caractérisé en ce que** les moyens d'irradiation de faisceaux lumineux objets (64; 106) comprennent
un système de lentilles avant (202) étant réglé de telle manière que les faisceaux lumineux objets (107a, 107b ; 107c) soient collimatés et se croisent à un point,
un lecteur de faisceaux optiques (206) étant placé à une position à laquelle les faisceaux lumineux objets (107a, 107b ; 107c) se croisent, et
un système de lentilles arrière (203, 204) qui amène les faisceaux lumineux objets (107a, 107b ; 107c) passant à travers le lecteur de faisceaux lumineux (206) à être condensés sur la cible de mesure, et
des changements, sur la cible de mesure (107a, 107b ; 107c), d'une position de chacun des faisceaux lumineux objets (107a, 107b ; 107c) avec lesquels différentes positions de la cible de mesure sont irradiées, et
dans lequel le dispositif de tomographie par cohérence optique comprend en outre
un mécanisme pour amener une lentille du système de lentilles arrière (203, 204) pour les moyens d'irradiation de faisceaux lumineux objets (64 ; 106) à se déplacer dans une direction parallèle à une direction de profondeur de la cible de mesure.

2. Dispositif de tomographie par cohérence optique selon la revendication 1, dans lequel
les moyens de génération de données de spectre optique (68, 109) génèrent des informations relatives à un changement d'un rapport d'intensité entre une première lumière d'interférence (R51, R52 ; R53) acquise et une deuxième lumière d'interférence (R61, 562 ; R63) acquise, et
le dispositif de tomographie par cohérence optique comprend en outre des moyens de commande pour acquérir des données structurelles dans la direction de profondeur en ce qui concerne la cible de mesure, sur la base des informations générées.

3. Procédé de génération d'une image tomographique par cohérence optique par un dispositif de tomographie par cohérence optique, comprenant :
la division d'une lumière émise depuis une source de lumière laser à balayage de longueur d'onde unique (101) en au moins quatre faisceaux divisés et la délivrance des faisceaux divisés ;
l'irradiation de différentes positions d'une cible de mesure avec des faisceaux lumineux objets (107a, 107b ; 107c) étant au moins deux parmi les au moins quatre faisceaux divisés par l'ajustement d'une position de chacun des faisceaux lumineux objets (107a, 107b; 107c) sur la cible de mesure, et également l'irradiation d'un miroir de faisceaux de référence (66 ; 108) avec au moins deux parmi les au moins quatre faisceaux divisés qui ne sont pas les faisceaux lumineux objets (107a, 107b ; 107c), en tant que faisceaux de référence (R41, R42 ; R43) ; et
l'acquisition de données structurelles dans la direction de profondeur en ce qui concerne la cible de mesure depuis une lumière d'interférence (R51, R52, R61, R62 ; R53, R63) à acquérir en amenant chacun des faisceaux de référence (R41, R42 ; R43) réfléchis par le miroir de faisceaux de référence (66 ; 108) à interférer avec l'un correspondant des faisceaux lumineux objets (R31, R32 ; R33) réfléchis ou diffusés par la cible de mesure,
**caractérisé en ce que** le dispositif de tomographie par cohérence optique comprend
un système de lentilles avant (202) étant réglé de telle manière que les faisceaux lumineux objets (107a, 107b ; 107c) soient collimatés et se croisent à un point,
un lecteur de faisceaux optiques (206) étant placé à une position à laquelle les faisceaux lumineux objets (107a, 107b ; 107c) se croisent, et
un système de lentilles arrière (203, 204) qui amène les faisceaux lumineux objets (107a, 107b ; 107c) passant à travers le lecteur de faisceaux lumineux (206) à être condensés sur la cible de mesure, et
le procédé comprend en outre le changement, sur la cible de mesure, d'une position de chacun des faisceaux lumineux objets (107a, 107b ; 107c) avec lesquels différentes positions de la cible de mesure sont irradiées,
le procédé comprenant en outre
l'ajustement d'une distance entre des positions irradiées sur la cible de mesure avec les faisceaux lumineux objets (107a, 107b ; 107c) en amenant une lentille du système de lentilles arrière (203, 204) dans le dispositif de tomographie par cohérence optique à se déplacer dans une direction parallèle à une direction de profondeur de la cible de mesure.

4. Procédé de génération d'une image tomographique par cohérence optique selon la revendication 3, dans lequel
des informations relatives à un changement d'un rapport d'intensité entre une première lumière d'interférence (R51, R52 ; R53) acquise et une deuxième lumière d'interférence (R61, 562 ; R63) acquise sont générées, et
les données structurelles dans la direction de profondeur en ce qui concerne la cible de mesure sont acquises, sur la base des informations générées.
